# EUROPÄISCHE PATENTSCHRIFT

(11) **EP 2 371 293 B1**
(45) Veröffentlichungstag und Bekanntmachung des Hinweises auf die Patenterteilung: **19.03.2014**
(21) Anmeldenummer: 11160059.9
(22) Anmeldetag: 28.03.2011
(51) Int. Cl.: A61B 17/00, A61B 18/00, A61B 1/018, A61B 1/00, A61B 10/04

(54) **Hubvorrichtung zur Bewegung einer Sonde in einem medizinischen Instrument**
Lifting device for moving a probe in a medical instrument
Dispositif de levage pour le déplacement d'une sonde dans un instrument médical

(30) Priorität: 29.03.2010 DE 102010013309
(43) Veröffentlichungstag der Anmeldung: 05.10.2011
(73) Patentinhaber: Karl Storz GmbH & Co. KG, 78532 Tuttlingen (DE)
(72) Erfinder: Wimmer, Viktor, 78532, Tuttlingen (DE)

(56) Entgegenhaltungen:
- EP-A1- 1 554 974
- WO-A2-2007/136630
- JP-A- 2005 312 828
- US-A- 5 910 105
- US-A1- 2008 242 925
- US-A1- 2009 259 105

## Beschreibung

Die vorliegende Erfindung bezieht sich auf eine Hubvorrichtung zur kontrollierten longitudinalen Bewegung einer Sonde in einem Arbeitskanal eines medizinischen Instruments, beispielsweise eines Endoskops, und auf ein Verfahren zum Vorbereiten einer Hubvorrichtung für eine Verwendung.

Starre und flexible Endoskope für medizinische oder nicht medizinische Anwendungen (in letzterem Fall auch als Boreskope bezeichnet) weisen oft einen oder mehrere Arbeitskanäle auf. Durch einen Arbeitskanal kann ein Fluid in den untersuchten oder behandelten Hohlraum eingeleitet oder aus diesem abgesaugt werden. Ferner kann durch einen Arbeitskanal eine Sonde bis zum distalen Ende des Endoskops und darüber hinaus vorgeschoben werden, während das Endoskop unverändert liegen bleiben kann. Weitere Beispiele für medizinische Instrumente mit Arbeitskanälen sind Rohrschaftinstrumente und Katheter.

Beispiele für Sonden sind medizinische Instrumente zur Diagnose oder Behandlung, wie etwa Zangen, Scheren oder Führungsdrähte. Ein weiteres Beispiel für eine Sonde ist ein Lichtwellenleiter, mittels dessen Laserlicht oder anderes Licht zum distalen Ende des Endoskops übertragen werden kann.

Eine Sonde kann in der Regel nicht nur einmalig in den Arbeitskanal des Endoskops eingeführt werden, sondern darin longitudinal bzw. in ihrer Längsrichtung beliebig hin- und herbewegt werden. In vielen Fällen ist eine genaue Positionierung des distalen Endes der Sonde relativ zum distalen Ende des Endoskops erforderlich, beispielsweise um eine Verletzung des Patienten durch das distale Ende der Sonde und/oder eine Beschädigung des distalen Endes der Sonde zu vermeiden. Ferner kann eine genaue Positionierung erforderlich sein, um eine definierte bzw. vorbestimmte chirurgische oder andere Wirkung der Sonde zu erzielen.

In der EP 1 554 974 A1 ist ein endoskopisch-chirurgisches Instrument beschrieben, das in einen Kanal eines Endoskops eingesetzt werden kann. Das Instrument weist am proximalen Ende einen Manipulationsabschnitt auf, mittels dessen das Instrument gedreht, vorgeschoben und zurückgezogen werden kann.

In der WO 2007/136630 A2 ist eine Endoskop-Werkzeug-Kupplung beschrieben, die die Anordnung und Positionierung eines chirurgischen Werkzeugs innerhalb eines Endoskops vereinfacht. Ein Kupplungskörper und eine Werkzeugaufnahme sind teleskopisch gegeneinander verschiebbar. Ein Eingriffs-Bauglied mit einer Mehrzahl von Zähnen, das mit dem Kupplungskörper verbunden ist, kann in eine Zahnstange an der Werkzeugaufnahme eingreifen, um die Werkzeugaufnahme in einer ausgewählten Position zu verriegeln.

Die US 5,910,105 beschreibt ein Instrument zum Führen einer endoskopischen Nadel, das auf einen Arbeitskanaleinang eines Endoskops montiert werden kann.

In der US 2008/024925 A1 ist ein Instrument beschrieben, das in einen Arbeitskanal eines Endoskops eingesetzt werden kann. Ein proximaler Abschnitt des Instruments kann gegen einen distalen Abschnitt linear verschoben und über einen Stopper in einer gewählten Position fixiert werden.

Die Aufgabe der vorliegenden Erfindung besteht darin, eine verbesserte Hubvorrichtung zur kontrollierten longitudinalen Bewegung einer Sonde in einem Arbeitskanal eines medizinischen Instrumentsund ein verbessertes Verfahren zum Vorbereiten einer Hubvorrichtung für eine Verwendung zu schaffen.

Diese Aufgabe wird durch die Gegenstände der unabhängigen Ansprüche gelöst.

Weiterbildungen sind in den abhängigen Ansprüchen angegeben.

Verschiedene Ausführungsformen der vorliegenden Erfindung beruhen auf der Idee, eine Hubvorrichtung zur kontrollierten longitudinalen Bewegung einer Sonde in einem Arbeitskanal eines medizinisches Instruments zu schaffen, die ein distales Bauteil mit einer Kopplungseinrichtung zum Befestigen der Hubvorrichtung am proximalen Ende des Arbeitskanals des medizinisches Instruments und ein proximales Bauteil mit einer Halteeinrichtung zum Halten eines Abschnitts der Sonde aufweist, wobei das proximale Bauteil relativ zu dem distalen Bauteil linear verschiebbar ist und die Position der Halteinrichtung relativ zur Kopplungseinrichtung stufenlos einstellbar ist, und wobei die Hubvorrichtung eine Verbindungseinrichtung aufweist, mittels der das proximale Bauteil mit dem distalen Bauteil lösbar verbunden ist, wobei die Verbindungseinrichtung einen ersten Zustand aufweist, in dem das proximale Bauteil verdrehsicher an dem distalen Bauteil festgelegt ist. Zur stufenlosen Einstellbarkeit ist beispielsweise ein O-Ring oder ein anderes ringförmiges Reibelement zwischen dem proximalen Bauteil und dem distalen Bauteil vorgesehen, um einer relativen linearen Verschiebung der beiden eine Reibungskraft entgegenzusetzen. Dabei sind die aneinander reibenden Oberflächen und ihre Eigenschaften und insbesondere das Reibelement so ausgebildet, dass die Haftreibung nicht oder nicht wesentlich größer ist als die Gleitreibung. Dadurch können gleichzeitig eine unbeabsichtigte Verstellung vermieden und eine feinfühlige Justage ermöglicht werden.

Ein Vorteil dieser Hubvorrichtung besteht je nach Anwendung der Hubvorrichtung darin, dass die Sonde in longitudinaler Richtung in jede beliebige Position und nicht nur in durch Zähne einer Zahnstange vorgegebene Positionen gebracht werden kann. Ferner ist die Verstellung der Hubvorrichtung und der Position der Sonde ohne ein vorheriges Entriegeln möglich. Medizinisches Personal kann deshalb die Sonde schneller, einfacher und genauer positionieren. Die lösbare Verbindungseinrichtung ermöglichst es einerseits, das proximale und das distale Bauteil sicher und verlässlich miteinander zu verbinden und ermöglicht es andererseits, diese Bauteile je nach Bedarf teilweise oder ganz voneinander zu lösen.

Darüber hinaus hat sie den Vorteil, dass die Sonde im ersten Zustand der Verbindungseinrichtung sicher in longitudinaler Richtung geführt werden und mit Betätigung der Hubvorrichtung vor- und zurückgeschoben werden kann, ohne sich dabei zu drehen. Durch die Verdrehsicherung des proximalen Bauteils wird ein unerwünschtes Verdrehen der Sonde erfolgreich verhindert.

Die Verbindungseinrichtung der Hubvorrichtung kann in einer weiteren vorteilhaften Ausgestaltung außerdem einen zweiten Zustand aufweisen, in dem das proximale Bauteil mit dem distalen Bauteil verbunden und gegenüber diesem verdrehbar ist. In diesem zweiten Zustand verhindert die Verbindung zwischen distalem und proximalem Bauteil, dass diese beiden Bauteile bei Betätigung des Hubmechanismus versehentlich voneinander getrennt werden. Gleichzeitig kann das proximale Bauteil mit der in der Halteeinrichtung gehaltenen Sonde um seine Längsachse gedreht werden, so dass die Sonde nach Belieben orientiert werden kann. Das distale Bauteil verbleibt dabei sicher auf dem proximalen Ende des Arbeitskanals des medizinischen Instruments.

In einer weiteren günstigen Ausgestaltung der Erfindung kann die Verbindungseinrichtung auch einen dritten Zustand aufweisen, in dem das proximale Bauteil vom distalen Bauteil trennbar ist. Die Trennbarkeit des proximalen Bauteils vom distalen Bauteil kann eine vollständige Reinigung und Sterilisation der Hubvorrichtung erleichtern. Durch die vollständige Zerlegbarkeit wird es möglich, die Bauteile getrennt voneinander zu reinigen.

Die Verbindungseinrichtung kann vorzugsweise eine Schraubenmutter umfassen. Die Schraubenmutter ist beispielsweise eine Überwurfmutter, die entlang des einen Bauteils innerhalb eines beidseitig begrenzten Intervalls verschiebbar und mit einem Gegengewinde am anderen Bauteil verschraubbar ist. Eine derartige Schraubenmutter kann eine mechanische Verbindung zwischen den beiden Bauteilen schaffen, die einerseits hinreichend fest und andererseits jederzeit mit geringem Aufwand lösbar ist.

Das distale Bauteil kann darüber hinaus ein erstes Rohr und das proximale Bauteil kann ein zweites Rohr umfassen, wobei das erste Rohr und das zweite Rohr eine Teleskoprohranordnung bilden. Diese Teleskoprohranordnung kann einerseits leicht herstellbar und andererseits besonders robust und leichtgängig sein.

Wenn das zweite Rohr einen nicht-kreisförmigen Querschnitt aufweist, insbesondere einen Querschnitt mit einer nicht-kreisförmigen äußeren Kontur, kann die Hubvorrichtung ein Führungselement aufweisen, das eine lineare Bewegung des zweiten Rohrs relativ zum Führungselement zulässt und eine Rotation des zweiten Rohrs relativ zum Führungselement formschlüssig unterbindet. Das Führungselement ist mit dem distalen Bauteil mechanisch verbunden oder verbindbar. Der nicht-kreisförmige Querschnitt weist hierfür eine dreieckige, quadratische, rechteckige oder andere polygonale Kontur auf. Das Führungselement ist insbesondere ausgebildet, um an der nicht-kreisförmigen Kontur teilweise oder vollständig anzuliegen. Das Führungselement ist beispielsweise ausgebildet, um den Querschnitt des zweiten Rohrs teilweise oder vollständig zu umfassen. Insbesondere weist das Führungselement eine Öffnung auf, deren Form an die äußere Kontur des Querschnitts des zweiten Rohrs angepasst ist und in der das zweite Rohr angeordnet ist.

Wenn das Führungselement nicht starr und unlösbar mit dem distalen Bauteil mechanisch verbunden ist, können das Führungselement, das distale Bauteil und die Schraubenmutter auf bevorzugte Weise so ausgebildet sein, dass bei arretierter und damit festgezogener Schraubenmutter, das Führungselement an einer Rotation gegenüber dem distalen Bauteil formschlüssig oder kraftschlüssig gehindert ist.

Somit ist es möglich, das zweite Rohr, wie bei der Verwendung der Hubvorrichtung vorgesehen, linear auf und ab zu bewegen und damit die von der Halteeinrichtung gehaltene Sonde linear zu verschieben. Dabei wird das zweite Rohr teilweise oder ganz vom Führungselement umschlossen und sicher geführt, so dass die lineare Bewegung möglich ist. Im ersten Zustand der Verbindungseinrichtung wird gleichzeitig eine unerwünschte Drehbewegung des zweiten Rohrs und damit der Sonde um die Längsachse zuverlässig verhindert. Die Betätigung des Hubmechanismus mit einer Hand ist damit leicht möglich.

Neben den oben genannten Vorteilen erleichtert ein lösbares Führungselement die Reinigung der Einzelteile der Hubvorrichtung.

Wenn das erste Rohr einen nicht-kreisförmigen Querschnitt aufweist, insbesondere einen Querschnitt mit einer nicht-kreisförmigen äußeren Kontur, kann die Hubvorrichtung ein Führungselement umfassen, das eine lineare Bewegung des ersten Rohrs relativ zum Führungselement zulässt und eine Rotation des ersten Rohrs relativ zum Führungselement formschlüssig unterbindet. Das Führungselement ist mit dem proximalen Bauteil verbunden oder verbindbar. Das erste Rohr weist hierfür einen Querschnitt mit einer dreieckigen, quadratischen, rechteckigen oder anderen polygonalen Kontur auf. Das Führungselement ist insbesondere ausgebildet, um an der nicht-kreisförmigen Kontur teilweise oder vollständig anzuliegen. Das Führungselement ist beispielsweise ausgebildet, um den Querschnitt des ersten Rohrs teilweise oder vollständig zu umfassen. Insbesondere weist das Führungselement eine Öffnung auf, deren Form an den Querschnitt des ersten Rohrs angepasst ist.

Wenn das Führungselement nicht starr und unlösbar mit dem proximalen Bauteil verbunden ist, können das Führungselement, das proximale Bauteil und die Schraubenmutter auf bevorzugte Weise so ausgebildet sein, dass bei arretierter und damit festgezogener Schraubenmutter das Führungselement an einer Rotation gegenüber dem proximalen Bauteil formschlüssig oder kraftschlüssig gehindert ist.

Ein Führungselement, wie es vorstehend beschrieben ist, kann eine relative Rotation des distalen Bauteils und des proximalen Bauteils unterbinden und gleichzeitig eine lineare Relativbewegung derselben ermöglichen. Damit kann eine unbeabsichtigte Rotation der Sonde verhindert werden. Dabei kann entweder das erste Rohr im zweiten Rohr oder das zweite Rohr im ersten Rohr angeordnet sein. Das Führungselement kann das zu führende Rohr sowohl an einer inneren Oberfläche als auch an einer äußeren Oberfläche berühren.

Eine Hubvorrichtung mit einer Teleskoprohranordnung, wie sie oben beschrieben ist, kann vorteilhafterweise einen O-Ring oder ein anderes ringförmiges Reibelement zur Erzeugung einer Reibungskraft bei einer Verschiebung des proximalen Bauteils relativ zum distalen Bauteil zwischen dem ersten Rohr und dem zweiten Rohr aufweisen. Das Reibelement ist insbesondere an einem äußeren Umfang desjenigen der beiden Rohre mit dem kleineren Querschnitt angeordnet. Beispielsweise setzt das beschriebene Reibelement zwischen dem ersten Rohr und dem zweiten Rohr auch einer Rotation der Rohre relativ zueinander eine Reibungskraft entgegen. Damit wird situationsabhängig auch eine kontrollierte Rotation der Sonde um ihre Längsachse ermöglicht.

Die Halteeinrichtung der Hubvorrichtung kann darüber hinaus ein oder mehrere Dichtungselemente, beispielsweise O-Ringe, und eine Kompressionseinrichtung zur Kompression des oder der Dichtungselemente umfassen. Der oder die Dichtungselemente sind insbesondere so angeordnet, dass sie einen Sondenkanal in der Hubvorrichtung umschließen. Durch eine Kompression des oder der Dichtungselemente in axialer Richtung werden diese in radialer Richtung verformt, wobei der von ihnen umschlossene Sondenkanal eingeengt wird. Diese Halteeinrichtung kann ein Halten eines im Wesentlichen zylindrischen Abschnitts einer Sonde in einer beliebig einstellbaren Position ermöglichen. Durch Variation der Kompression des oder der Dichtungselemente kann die Haltekraft bzw. die Reibungskraft zwischen dem Abschnitt der Sonde und dem oder den Dichtungselementen variiert werden. Die Kompressionseinrichtung kann insbesondere ein Schraubgewinde umfassen.

Auf diese Weise wird es dem Operateur ermöglicht, die Halteeinrichtung auf einfache Weise nach Bedarf zu lösen um die Sonde neu zu justieren. Bei geschlossener Kompressionseinrichtung wird die Sonde sicher gehalten und ein versehentliches Verschieben oder Verdrehen verhindert.

Ist das proximale Bauteil verdrehsicher am distalen Bauteil festgelegt, kann die Kompressionseinrichtung einfach mit einer Hand des Operateurs geöffnet und wieder verschlossen werden, ohne dass sich Teile der Hubvorrichtung unerwünschterweise mit bewegen.

Die Halteeinrichtung kann ferner zum fluiddichten Verschließen des proximalen Endes der Hubvorrichtung mit und ohne eingesetzte Sonde ausgebildet sein. Dazu sind beispielsweise die O-Ringe durch axiale Kompression so weit verformbar, dass sie den Sondenkanal vollständig verschließen. Die Hubvorrichtung kann in diesem Fall mit einer Spülflüssigkeit oder einem anderen Fluid gefüllt oder gespült werden, ohne dass dieses Fluid am proximalen Ende der Hubvorrichtung austräte. Auch mit eingesetzter Sonde wird diese so dicht von der Halteeinrichtung umschlossen, dass die Zuführung von Spülflüssigkeit in die Hubvorrichtung und den Arbeitskanal möglich ist, ohne dass proximal Flüssigkeit austritt. So ist während der Verwendung der Hubvorrichtung ein Spülen des zu behandelnden Gebiets am distalen Ende des Instruments möglich.

Die Hubvorrichtung kann insbesondere zur kontrollierten Bewegung eines Lichtwellenleiters, beispielsweise einer oder mehrerer Glasfasern, ausgebildet sein. Dazu sind insbesondere die Querschnitte eines Sondenkanals in der Hubvorrichtung und die Halteeinrichtung an einen Lichtwellenleiter angepasst. Gerade die genaue Positionierung eines Lichtwellenleiters, z.B. einer Laserfaser, in einem Endoskop kann wichtig sein, um beispielsweise eine Beschädigung oder Zerstörung des Endoskops durch am distalen Ende des Lichtwellenleiters austretende Laserstrahlung zu verhindern. Eine kontrollierte longitudinale Bewegung, insbesondere eine genaue Positionierung einer Sonde mit einer der oben beschriebenen Hubvorrichtungen ermöglicht ferner einen Schutz des distalen Endes der Sonde einerseits und des Patienten andererseits, beispielsweise beim Einführen des Endoskops, durch ein definiertes Zurückziehen des distalen Endes der Sonde in das distale Ende des Endoskops.

Die Hubvorrichtung kann in einer weiteren Ausgestaltung mit einem medizinischen Instrument verbunden sein und am distalen Ende der Kopplungseinrichtung eine nicht-runde Aufnahme aufweisen, so dass die Hubvorrichtung verdrehsicher auf einem zur nicht-runden Aufnahme korrespondierenden Ausgang des Arbeitskanals des medizinischen Instruments gelagert werden kann. Dies ermöglichst es vorteilhafterweise, die Hubvorrichtung mit einer Hand zu betätigen, beispielsweise das proximale Bauteil gegen das distale Bauteil zur Positionierung der Sonde zu verdrehen oder die Kompressionseinrichtung zu öffnen und zu schließen, ohne dabei die Hubvorrichtung als Ganzes mit zu verdrehen. Der zur nicht-runden Aufnahme passende Arbeitskanalausgang des medizinischen Instruments dient dabei als eine Art Widerlager.

Die Hubvorrichtung ist sowohl für starre als auch für flexible Instrumente geeignet.

Verschiedene Ausführungsformen der vorliegenden Erfindung beruhen ferner auf der Idee, bei einem Verfahren zum Vorbereiten einer Hubvorrichtung mit einem distalen Bauteil und einem proximalen Bauteil für eine Verwendung eine Schraubenmutter zu lösen, das distale Bauteil von dem proximalen Bauteil zu trennen, das distale Bauteil und das proximale Bauteil getrennt zu reinigen, das distale Bauteil und das proximale Bauteil nach dem Reinigen wieder zusammenzusetzen und die Schraubenmutter festzuziehen, um das distale Bauteil und das proximale Bauteil zu verbinden. Das Trennen der Bauteile bzw. das Zerlegen der Hubvorrichtung nach dem Lösen der Schraubenmutter und vor dem Reinigen ermöglicht eine besonders umfassende und sichere Reinigung der Hubvorrichtung. Die Reinigung kann dabei auch eine Sterilisation der Hubvorrichtung umfassen. Durch Lösen bzw. Festziehen der Schraubenmutter können die Bauteile der Hubvorrichtung besonders einfach voneinander getrennt bzw. miteinander mechanisch verbunden werden.

Vor dem Reinigen der getrennten Bauteile, insbesondere vor dem Reinigen des proximalen Bauteils kann ferner eine Kompressionseinrichtung oder ein Teil einer Kompressionseinrichtung von einer Halteeinrichtung am proximalen Bauteil getrennt werden, die nach dem Reinigen wieder am proximalen Bauteil angeordnet wird.

### Kurzbeschreibung der Figuren

Nachfolgend werden beispielhafte Ausführungsformen anhand der beigefügten Figuren näher erläutert. Es zeigen:
- Figur 1: schematische Darstellungen eines Endoskops, einer Hubvorrichtung und einer Sonde in zwei verschiedenen Zuständen;
- Figur 2: schematische Darstellungen einer Hubvorrichtung in zwei verschiedenen Zuständen;
- Figur 3: schematische Darstellungen von Teilen einer Hubvorrichtung;
- Figur 4: schematische Darstellungen von Teilen einer Hubvorrichtung;
- Figur 5: ein schematisches Flussdiagramm eines Verfahrens zum Vorbereiten einer Hubvorrichtung.

### Beschreibung der Ausführungsformen

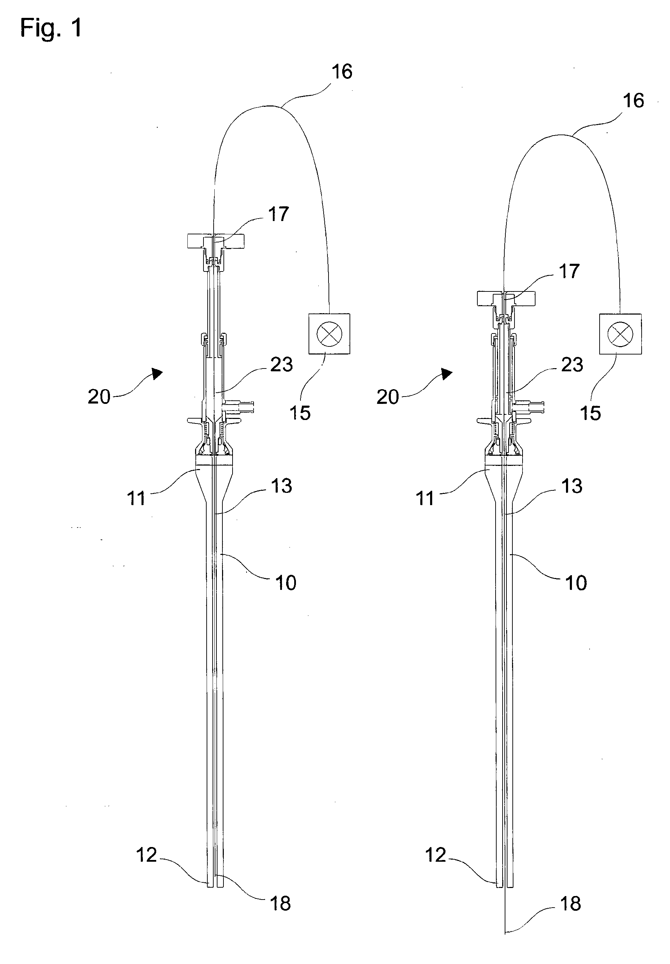

Figur 1 zeigt schematische Darstellungen eines medizinischen Instruments, nämlich eines Endoskops 10 mit einem proximalen Ende 11, einem distalen Ende 12 und einem Arbeitskanal 13, der sich vom proximalen Ende 11 zum distalen Ende 12 des Endoskops 10 erstreckt. Mit dem proximalen Ende 11 des Endoskops 10 ist eine Hubvorrichtung 20 verbunden. Die Hubvorrichtung weist einen Sondenkanal 23 auf, dessen distales Ende mit dem proximalen Ende des Arbeitskanals 13 verbunden oder zumindest ausgerichtet ist. Die Figur 1 zeigt das Endoskop 10 und die Hubvorrichtung 20 jeweils in einer Schnittdarstellung, wobei die Schnittebene eine Längsachse des Arbeitskanals 13 enthält.

Eine Lichtquelle 15 ist mit einem Lichtwellenleiter 16 gekoppelt, der zum Leiten von Licht, das von der Lichtquelle 15 erzeugt wird, ausgebildet und vorgesehen ist. Der Lichtwellenleiter 16 ist teilweise in dem Arbeitskanal 13 des Endoskops 10 und in dem Sondenkanal 23 der Hubvorrichtung 20 angeordnet. Ein distales Ende 18 des Lichtwellenleiters 16 ist nahe am distalen Ende 12 des Endoskops 10 angeordnet. Ein proximaler Abschnitt 17 des Lichtwellenleiters 16 ist an einem proximalen Ende der Hubvorrichtung 20 gehalten. Der proximale Abschnitt 17 wird in Abgrenzung zum distalen Ende 18 des Lichtwellenleiters 16 als proximal bezeichnet, obwohl das proximale Ende des Lichtwellenleiters 16, das mit der Lichtquelle 15 gekoppelt ist, von dem proximalen Abschnitt 17 beabstandet sein kann, wie es beispielsweise auch in Figur 1 dargestellt ist.

Die beiden Darstellungen des Endoskops 10, der Hubvorrichtung 20 und des Lichtwellenleiters 16 unterscheiden sich in der Konfiguration bzw. im Zustand der Hubvorrichtung 20. Bei der Darstellung links ist die Hubvorrichtung 20 gestreckt, bei der Darstellung rechts in Figur 1 ist sie verkürzt bzw. zusammen geschoben gezeigt. Da der proximale Abschnitt 17 des Lichtwellenleiters 16 am proximalen Ende der Hubvorrichtung 20 gehalten ist, wird das distale Ende 18 des Lichtwellenleiters 16 beim Verkürzen bzw. Zusammenschieben der Hubvorrichtung 20 nach distal geschoben und beim Auseinanderziehen bzw. Strecken der Hubvorrichtung 20 nach proximal gezogen. Deshalb liegt das distale Ende 18 des Lichtwellenleiters 16 bei der Darstellung links in Figur 1 im Arbeitskanal 13 des Endoskops 10, während es bei der Darstellung rechts in Figur 1 gegenüber dem distalen Ende 12 des Endoskops 10 übersteht.

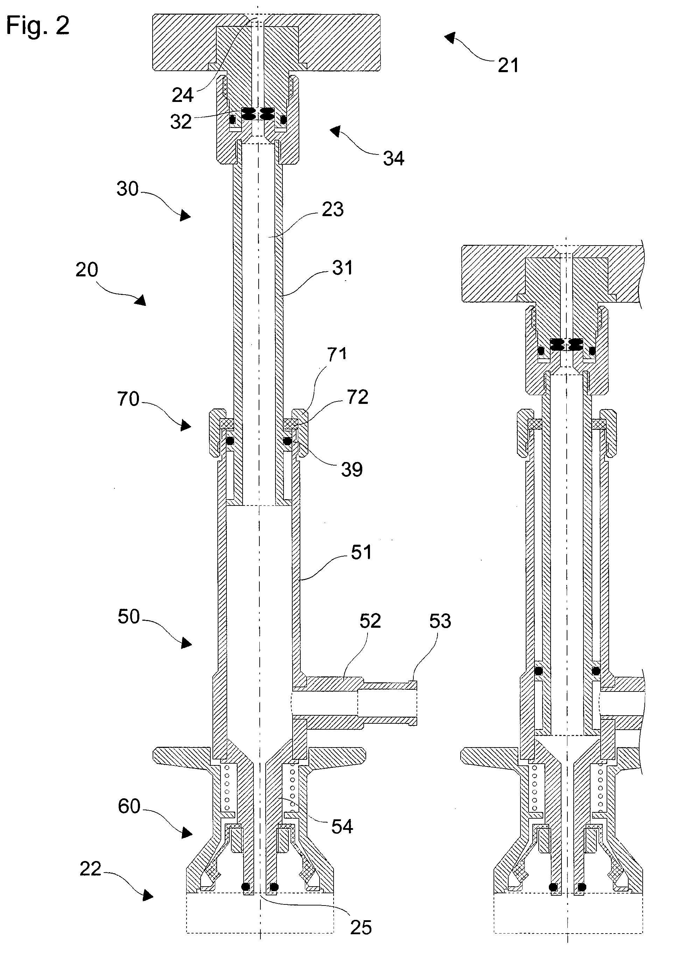

Figur 2 zeigt schematische Darstellungen der Hubvorrichtung 20 in den beiden in Figur 1 gezeigten Konfigurationen bzw. Stellungen. Vom proximalen Ende 21 bis zum distalen Ende 22 der Hubvorrichtung 20 erstreckt sich der bereits oben erwähnte Arbeitskanal 23. Der Arbeitskanal 23 ist sowohl an seinem proximalen Ende 24 als auch an seinem distalen Ende 25 offen.

Die Hubvorrichtung 20 umfasst ein proximales Bauteil 30 mit einem Rohr 31. Das Rohr 31 umfasst ferner O-Ringe 32 und eine Kompressionseinrichtung 34 für die O-Ringe 32 am proximalen Ende 21 der Hubvorrichtung. Die O-Ringe 32 und die Kompressionseinrichtung 34 bilden eine Halteeinrichtung, die weiter unten näher beschrieben wird. Am distalen Ende des Rohrs 31 des proximalen Bauteils ist ein O-Ring 39 vorgesehen, dessen Funktion unten beschrieben wird.

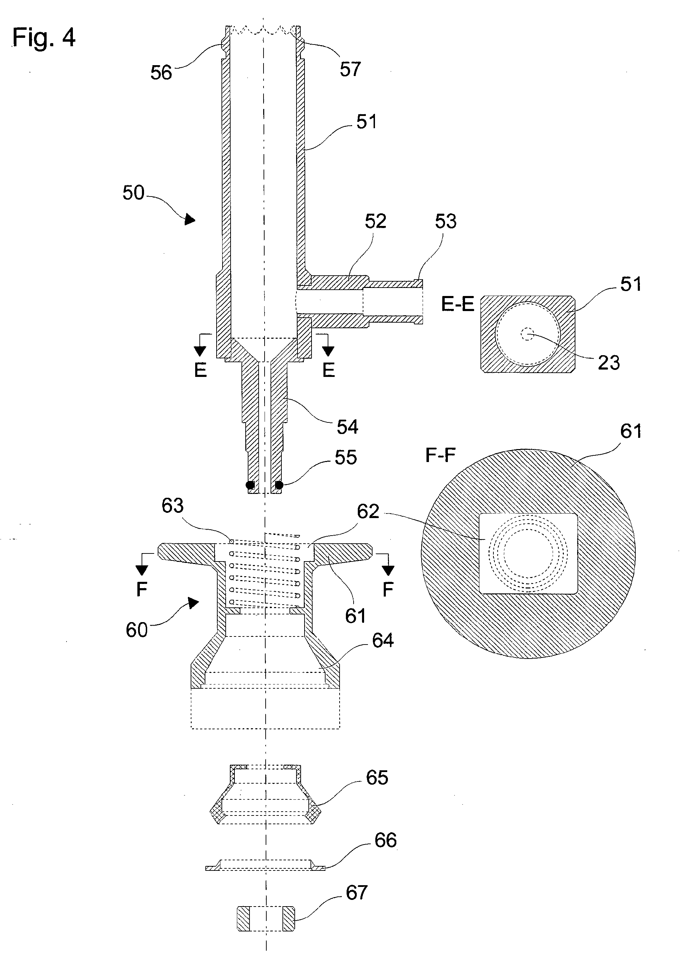

Die Hubvorrichtung 20 umfasst ferner ein distales Bauteil 50 mit einem Rohr 51. Nahe dem distalen Ende des Rohrs 51 ist ein Spülanschlussstutzen 52 mit einer Kupplung 53, beispielsweise einer Luer-Lock-Kupplung, angeordnet. Das Rohr 51 geht an seinem distalen Ende in ein distales Anschlussrohr 54 über. Das distale Ende des Rohrs 51 ist ferner mit einer Kopplungseinrichtung 60 verbunden, die unten anhand der Figur 4 näher beschrieben wird.

Am proximalen Ende des Rohrs 51 des distalen Bauteils 50 ist eine Verbindungseinrichtung 70 zur mechanischen Verbindung des proximalen Bauteils 30 und des distalen Bauteils 50 angeordnet. Die Verbindungseinrichtung 70 umfasst eine Schraubenmutter 71 in Form einer Überwurfmutter und ein Führungselement 72, das von der Schraubenmutter 71 am proximalen Ende des Rohrs 51 des distalen Bauteils 50 gehalten wird.

Die arretierte Schraubenmutter 71 stellt einen ersten Zustand der Verbindungseinrichtung 70 dar, in dem proximales Bauteil 30 und distales Bauteil 50 verdrehsicher aneinander festgelegt sind. Löst der Operateur die Schraubenmutter 71 teilweise, bringt er die Verbindungseinrichtung 70 in den zweiten Zustand, in dem proximales Bauteil 30 und distales Bauteil 50 miteinander verbunden, aber gegeneinander verdrehbar sind. Ein vollständiges Lösen der Schraubenmutter ermöglicht schließlich in einem dritten Zustand das vollständige Trennen des proximalen Bauteils 30 vom distalen Bauteil 50.

Das proximale Bauteil 30 mit der aus den O-Ringen 32 und der Kompressionseinrichtung 34 gebildeten Halteeinrichtung einerseits und das distale Bauteil 50 mit dem Spülanschlussstutzen 52 und der Kopplungseinrichtung 60 andererseits sind relativ zu einander linear verschiebbar. Das Rohr 31 des proximalen Bauteils 30 und das Rohr 51 des distalen Bauteils 50 bilden dabei eine Teleskoprohranordnung. Der O-Ring 39 zwischen dem Rohr 31 des proximalen Bauteils 30 und dem Rohr 51 des distalen Bauteils 50 setzt einer Verschiebung des proximalen Bauteils 30 und des distalen Bauteils 50 relativ zueinander eine Reibungskraft entgegen. Die Geometrie und das Material des O-Rings 39 und das Material und vor allem die Beschaffenheit der inneren Oberfläche des Rohrs 51 des distalen Bauteils 50 sind so gewählt, dass die Reibung ein versehentliches Verstellen bzw. eine versehentliche relative Verschiebung des proximalen Bauteils 30 und des distalen Bauteils 50 möglichst zuverlässig verhindert und gleichzeitig eine gewollte Verschiebung in möglichst feinfühliger Weise ermöglicht wird. Dazu ist die Haftreibung insbesondere nicht oder nicht wesentlich größer als die Gleitreibung.

Links in Figur 2 ist die Hubvorrichtung 20 in einem expandierten bzw. gestreckten bzw. ausgefahrenen Zustand gezeigt, bei dem das proximale Ende 21 mit den O-Ringen 32 und der Kompressionseinrichtung 34 einerseits und das distale Ende 22 mit der Kopplungseinrichtung 60 andererseits einen maximalen Abstand voneinander haben. Rechts in Figur 2 ist die Hubvorrichtung 20 in einem zusammen geschobenen bzw. verkürzten Zustand gezeigt, bei dem das proximale Ende 21 mit den O-Ringen 32 und der Kompressionseinrichtung 34 einerseits und das distale Ende 22 mit der Kopplungseinrichtung 60 andererseits einen minimalen Abstand voneinander aufweisen.

Wenn das distale Ende 22 der Hubvorrichtung 20 mittels der Kopplungseinrichtung 60 wie in Figur 1 dargestellt mit dem proximalen Ende 11 eines Endoskops 10 gekoppelt bzw. mechanisch verbunden ist, und wenn ein proximaler Abschnitt einer Sonde am proximalen Ende 21 der Hubvorrichtung gehalten wird (insbesondere durch die O-Ringe 32), hat eine Bewegung der Hubvorrichtung zwischen den beiden in Figur 2 dargestellten Zuständen eine entsprechende Bewegung der Sonde in ihrer Längsrichtung zur Folge. Damit wird insbesondere auch das distale Ende der Sonde in Längsrichtung zwischen einer proximalen Position und einer distalen Position hin- und herbewegt, wie dies bereits oben anhand der Figur 1 dargestellt wurde.

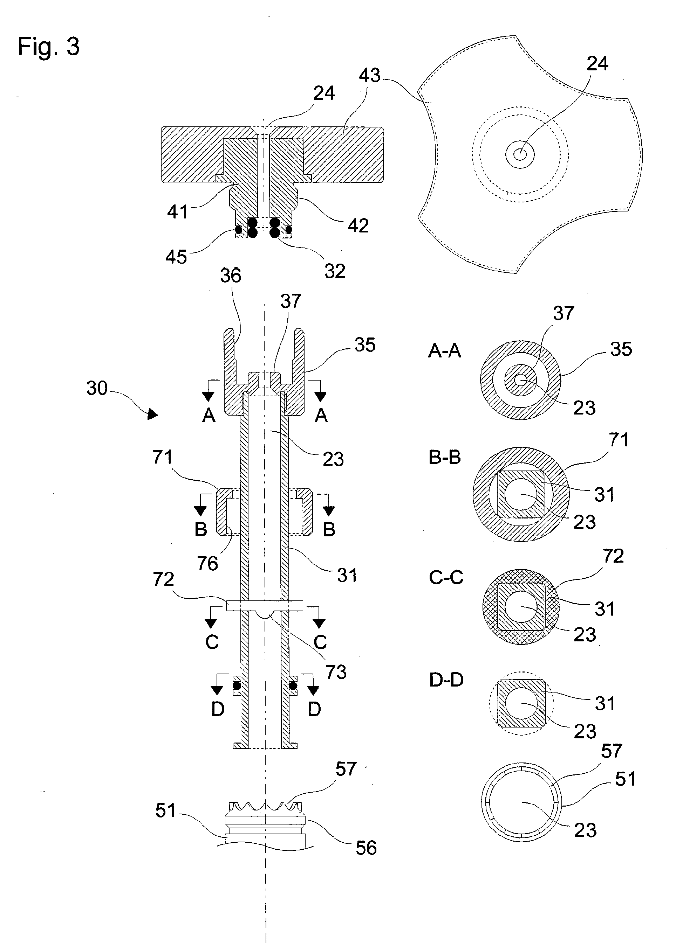

Figur 3 zeigt schematische Darstellungen von Bestandteilen der Hubvorrichtung 20, die oben anhand der Figuren 1 und 2 dargestellt wurde. Dabei ist links jeweils ein Schnitt entlang einer Ebene, welche die Längsachse des Sondenkanals 23 enthält, dargestellt. Rechts sind jeweils Draufsichten (ganz oben und ganz unten) bzw. Schnitte gezeigt, bei denen die Zeichenebene bzw. die Schnittebene A-A, B-B, C-C bzw. D-D senkrecht zur Längsachse des Sondenkanals liegt.

Die oben bereits erwähnte Kompressionseinrichtung 34 umfasst eine Schraubhülse 35 am proximalen Bauteil 30. Die Schraubhülse 35 ist mit dem proximalen Ende des Rohrs 31 des proximalen Bauteils 30 beispielsweise verschraubt und/oder verschweißt. Die Schraubhülse 35 weist ein Innengewinde 36 und einen kreisringförmigen Stempel 37 auf.

Die Kompressionseinrichtung 34 umfasst ferner ein Gewindebauteil 41 mit einem Außengewinde 42, einem Drehgriff 43 und einem O-Ring 45. Während das Gewindebauteil 41 beispielsweise Metall aufweist, kann der Drehgriff 43 ein anderes Material aufweisen, beispielsweise Kunststoff. In diesem Fall sind das Gewindebauteil 41 und der Drehgriff 43 miteinander verschweißt, verlötet, verklebt, verrastet oder auf andere Weise stoff-, kraftoder formschlüssig miteinander verbunden.

Das Außengewinde 42 des Gewindebauteils 41 kann in das Innengewinde 36 der Schraubhülse 35 geschraubt werden. Eine Rotation des Drehgriffs 43 mit dem Gewindebauteil 41 relativ zur Schraubhülse 35 hat dann gleichzeitig eine axiale Verschiebung des Gewindebauteils 41 und des Stempels 37 zur Folge. Dadurch können die O-Ringe 32, wie in Figur 2 angedeutet, in axialer Richtung gestaucht bzw. komprimiert werden. Dies hat eine Verformung der O-Ringe 32 in radialer Richtung zur Folge, die in Figur 2 ebenfalls angedeutet ist und eine lokale Verengung des Sondenkanals 23 bewirkt. Wenn eine Sonde sich im Sondenkanal 23 befindet, drücken die O-Ringe 32 in radialer Richtung auf die äußere Oberfläche der Sonde. Es entsteht eine Reibungskraft, die einer Verschiebung der Sonde in Längsrichtung oder einer Rotation der Sonde entgegenwirkt.

Durch eine Rotation des Drehgriffs 43 mit dem Gewindebauteil 41 können die axiale Kompression und die resultierende radiale Verformung der O-Ringe 32, der radiale Druck der O-Ringe 32 auf eine Sonde im Sondenkanal 23 und die Reibungskraft zwischen den O-Ringen 32 und der Sonde eingestellt werden. Reibung zwischen dem O-Ring 45, dem Gewindebauteil 41 und der Schraubhülse 35 verringert ein Risiko eines unbeabsichtigten Verdrehens des Drehgriffs 43 und Verstellens der Kompressionseinrichtung 34. Die O-Ringe 32 können so ausgebildet sein, dass sie bei großer axialer Kompression den Sondenkanal mit und ohne eingeführte Sonde vollständig und fluiddicht verschließen.

Während links in Figur 3 die meisten Teile in einem Schnitt entlang einer Ebene parallel zur Längsachse des Sondenkanals 23 dargestellt sind, ist das Führungselement 72 links in einer Seitenansicht gezeigt, in der eine Nase 73 erkennbar ist. Der rechts gezeigte Schnitt nach C-C zeigt, dass das Rohr 31 des proximalen Bauteils 30 zwischen der Schraubhülse 35 und dem O-Ring 39 einen Querschnitt mit einer im Wesentlichen quadratischen äußeren Kontur aufweist. Das Führungselement 72 ist von der Nase 73 abgesehen im Wesentlichen scheibenförmig mit einem kreisförmigen äußeren Rand. Das Führungselement 72 weist eine Öffnung bzw. ein Loch bzw. eine Ausnehmung auf, deren Form an die äußere Kontur des Querschnitts des Rohrs 71 angepasst ist. Eine relative Rotation des Führungselements 72 und des Rohrs 31 ist dadurch auf ein kleines Spiel reduziert.

Links in Figur 3 ganz unten ist das proximale Ende des Rohrs 51 des distalen Bauteils 50 der Hubvorrichtung 20 gezeigt, und zwar ähnlich wie das Führungselement 72 nicht in einer Schnittdarstellung sondern in einer Seitenansicht. Das Rohr 51 weist am proximalen Ende ein Gewinde 56 und einen Zahnkranz 57 auf. Die Nase 73 oder auch mehrere entsprechende Nasen am Führungselement 72 einerseits und der Zahnkranz 57 am proximalen Ende des Rohrs 51 andererseits sind geometrisch aneinander angepasst.

Die Schraubenmutter 71 weist ein Innengewinde 76 auf, das zum Gewinde 56 am proximalen Ende des Rohrs 51 des distalen Bauteils 50 komplementär ist. Wenn die Schraubenmutter 71 an das proximale Ende des Rohrs 51 geschraubt wird, liegt das Führungselement 72 wie in Figur 2 gezeigt zwischen der Schraubenmutter 71 und dem Zahnkranz 57. Wird die Schraubenmutter 71 vollständig arretiert, greifen die Nase oder die Nasen 73 so in den Zahnkranz 57 ein, dass eine Rotation des Führungselements 72 relativ zum Rohr 51 des distalen Bauteils 50 formschlüssig unterbunden wird.

Wenn die Schraubenmuter 71 so auf das Gewinde 56 am proximalen Ende des Rohrs 51 geschraubt ist, sind das proximale Bauteil 30 und das distale Bauteil 50 miteinander verbunden. Dabei kann aber das proximale Bauteil 30 gegenüber dem distalen Bauteil 50 innerhalb eines beidseitig begrenzten linearen Bereichs bewegt werden.

Nach teilweisem Lösen der Schraubenmutter 71 löst sich auch das Führungselement 72 vom Zahnkranz 57. Das Rohr 31 des proximalen Bauteils 30 ist dann noch mit dem Rohr 51 des distalen Bauteils 50 verbunden, kann jedoch gegen dieses verdreht werden, da die Nasen 73 nicht mehr in den Zahnkranz 57 eingreifen.

Nach vollständigem Lösen der Schraubenmutter 71 von dem Gewinde 56 kann das Rohr 31 des proximalen Bauteils 30 vollständig aus dem Rohr 51 des distalen Bauteils gezogen werden. Das proximale Bauteil 30 und das distale Bauteil 50 sind danach von einander getrennt und können in diesem getrennten Zustand beispielsweise gereinigt werden. Dabei sind beispielsweise die Innenwände der Rohre 31, 51 optimal zugänglich.

Figur 4 zeigt schematische Darstellungen von weiteren Bestandteilen der oben anhand der Figuren 1 und 2 dargestellten Hubvorrichtung. Gezeigt sind jeweils Schnitte entlang einer Ebene, die die Längsachse des Sondenkanals 23 enthält. Rechts in Figur 4 sind zwei Schnitte nach den links markierten Ebenen E-E und F-F gezeigt.

Das Rohr 51 des distalen Bauteils 50 weist, wie bereits oben anhand der Figur 3 dargestellt, an seinem proximalen Ende ein Gewinde 56 und einen Zahnkranz 57 auf. Der Spülanschlussstutzen 52 mit der Kupplung 53 und das distale Anschlussrohr 54 können jeweils einstückig mit dem Rohr 51 des distalen Bauteils 50 ausgebildet und aus einem einzigen Werkstück geformt oder wie in den Figuren 2 und 4 angedeutet, mit dem Rohr 51 verschraubt und/oder verschweißt, verlötet, verklebt oder auf andere Weise gefügt sein. Am distalen Ende des distalen Anschlussrohrs 54 ist ein O-Ring 55 angeordnet, der eine fluiddichte Verbindung mit einem Arbeitskanal eines Endoskops ermöglicht.

Die Kopplungseinrichtung 60 umfasst eine Griffplatte 61 und eine im Wesentlichen rechteckige Ausnehmung 62 am proximalen Ende sowie einen Innenkonus 64 am distalen Ende und eine Spiralfeder 63.

In den Innenkonus 64 ist eine Rasteinrichtung 65 einsetzbar. Ein ringförmiges Element 66 kann am distalen Rand des Innenkonus 64 durch Schweißen, Löten, Kleben oder auf andere Weise gefügt werden, um die Rasteinrichtung 65 im Innenkonus 64 zu halten.

Das distale Anschlussrohr 54 kann von proximal nach distal durch die Kopplungseinrichtung 60 geführt und mittels einer Schraubenmutter 67 mit der Rasteinrichtung 65 mechanisch verbunden werden. Das Rohr 51 weist an seinem distalen Ende einen rechteckigen Querschnitt auf, der dem Querschnitt der rechteckigen Ausnehmung 62 in der Kopplungseinrichtung entspricht, wie in der Schnittansicht entlang der Ebene E-E dargestellt. Das Rohr 51 ist deshalb gegenüber der Kopplungseinrichtung 60 axial verschiebbar aber nicht rotierbar.

Wenn das Rohr 51 und die Kopplungseinrichtung 60 gegen die Kraft der Spiralfeder 63 zusammengedrückt werden, wird die Rasteinrichtung 65 im Innenkonus 64 nach distal verschoben. Der segmentierte distale Rand der Rasteinrichtung 65 weicht radial nach außen aus, wodurch eine Verbindung mit einem proximalen Ende eines Endoskops hergestellt oder gelöst werden kann.

Eine am distalen Ende der Kopplungseinrichtung 60 angebrachte Aufnahme 68 ist nichtrund, beispielsweise viereckig, ausgestaltet und kann mit einem dazu komplementären Ausgang (14) des Arbeitskanals (11) des medizinischen Instruments verbunden werden. Auf diese Weise wird der Hubmechanismus (20) verdrehsicher auf dem medizinischen Instrument (10) gelagert. Wird nun der proximale Abschnitt (30) gegenüber dem distalen Abschnitt (50) verdreht, oder öffnet ein Operateur die Kompressionseinrichtung (34), um die Sonde (16) neu zu justieren, so verbleibt das distale Bauteil (50) lagerichtig auf dem medizinischen Instrument (10) ohne sich zu verdrehen. Dies ermöglicht die Bedienung des Hubmechanismus (20) mit einer Hand, da keine zweite Hand zur Fixierung des distalen Bauteils (50) notwendig ist.

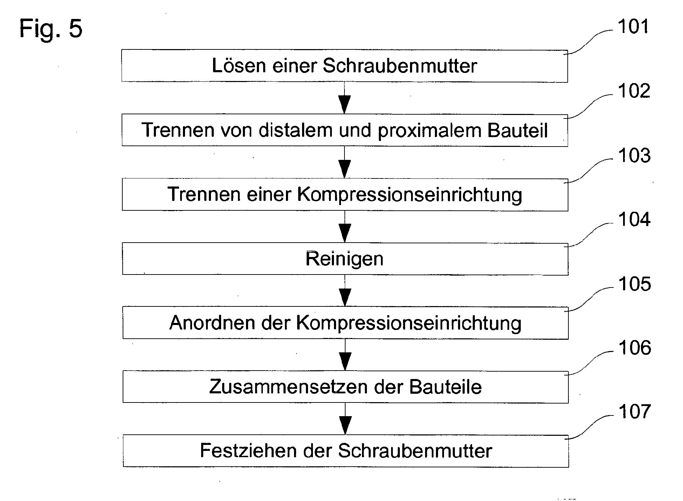

Figur 5 zeigt ein schematisches Flussdiagramm eines Verfahrens zum Vorbereiten einer Hubvorrichtung für eine Verwendung. Obwohl das Verfahren auch auf Hubvorrichtungen anwendbar ist, die sich von den oben anhand der Figuren 1 bis 4 dargestellten unterscheiden, werden nachfolgend zur Vereinfachung des Verständnisses Bezugszeichen aus den Figuren 1 bis 4 beispielhaft verwendet.

Bei einem ersten Schritt 101 wird eine Schraubenmutter 71 gelöst. Bei einem zweiten Schritt 102 wird ein distales Bauteil 50 von einem proximalen Bauteil 30 der Hubvorrichtung 20 getrennt, beispielsweise indem das proximale Bauteil 30 vollständig aus dem distalen Bauteil 50 gezogen wird. Bei einem optionalen dritten Schritt 103 wird eine Kompressionseinrichtung 34 oder ein Teil 41, 43 einer Kompressionseinrichtung vom proximalen Bauteil 30 getrennt.

Bei einem vierten Schritt 104 werden das proximale Bauteil 30, das distale Bauteil 50 und gegebenenfalls die Kompressionseinrichtung 34 oder ein Teil 41, 43 der Kompressionseinrichtung 34 getrennt voneinander gereinigt. Die getrennte Reinigung kann gleichzeitig und im selben Gefäß erfolgen und ermöglicht eine optimale Reinigung von Hohlräumen und Innenräumen, die anderenfalls nur schwer oder gar nicht zugänglich wären. Das Reinigen kann dabei auch ein Autoklavieren umfassen.

Bei einem optionalen fünften Schritt 105 wird gegebenenfalls die beim dritten Schritt 103 vom proximalen Bauteil 30 getrennte Kompressionseinrichtung 34 oder der beim dritten Schritt 103 vom proximalen Bauteil 30 getrennte Teil der Kompressionseinrichtung wieder am proximalen Bauteil 30 angeordnet und mit diesem verbunden. Bei einem sechsten Schritt 106 werden das proximale Bauteil 30 und das distale Bauteil 50 zusammengesetzt, insbesondere das proximale Bauteil 30 in das distale Bauteil 50 eingeführt. Bei einem siebten Schritt 107 wird die Schraubenmutter 71 festgezogen, um das proximale Bauteil 30 und das distale Bauteil 50 miteinander zu verbinden.

Der dritte Schritt 103 kann alternativ vor dem zweiten Schritt 102 oder vor dem ersten Schritt 101 ausgeführt werden. Der fünfte Schritt 105 kann alternativ nach dem sechsten Schritt 106 oder nach dem siebten Schritt 107 ausgeführt werden.

### Bezugszeichen

- 10: Endoskop
- 11: proximales Ende des Endoskops 10
- 12: distales Ende des Endoskops 10
- 13: Arbeitskanal des Endoskops 10
- 14: Ausgang
- 15: Lichtquelle
- 16: Lichtwellenleiter
- 17: proximaler Abschnitt des Lichtwellenleiters 15
- 18: distales Ende des Lichtwellenleiters 15
- 20: Hubvorrichtung
- 21: proximales Ende der Hubvorrichtung 20
- 22: distales Ende der Hubvorrichtung 20
- 23: Sondenkanal in der Hubvorrichtung 20
- 24: proximales Ende des Sondenkanals 23
- 25: distales Ende des Sondenkanals 23
- 30: proximales Bauteil der Hubvorrichtung 20
- 31: Rohr des proximalen Bauteils 30
- 32: O-Ring
- 34: Kompressionseinrichtung für den O-Ring 32
- 35: Schraubhülse des proximalen Bauteils 30
- 36: Innengewinde an der Schraubhülse 35
- 37: Stempel an der Schraubhülse 35
- 39: O-Ring am distalen Ende des Rohrs 31 des proximalen Bauteil 30
- 41: Gewindebauteil
- 42: Außengewinde am Gewindebauteil 41
- 43: Drehgriff
- 45: O-Ring an der Kompressionseinrichtung 40
- 50: distales Bauteil der Hubvorrichtung 20
- 51: Rohr des distalen Bauteils 50
- 52: Spülanschlussstutzen des distalen Bauteils 50
- 53: Kupplung am Spülanschlussstutzen 52
- 54: distales Anschlussrohr am distalen Bauteil 50
- 55: O-Ring am distalen Anschlussrohr 54
- 56: Gewinde am proximalen Ende des distalen Bauteils 50
- 57: Zahnkranz am proximalen Ende des distalen Bauteils 50
- 60: Kopplungseinrichtung
- 61: Griffplatte an der Kopplungseinrichtung 60
- 62: Ausnehmung in der Kopplungseinrichtung 60
- 63: Spiralfeder
- 64: Innenkonus
- 65: Rasteinrichtung
- 66: ringförmiges Element
- 67: Schraubenmutter
- 68: Aufnahme
- 70: Verbindungseinrichtung
- 71: Schraubenmutter der Verbindungseinrichtung 70
- 72: Führungselement
- 73: Nase am Führungselement
- 76: Innengewinde an der Schraubenmutter 71
- 101: erster Schritt
- 102: zweiter Schritt
- 103: dritter Schritt
- 104: vierter Schritt
- 105: fünfter Schritt
- 106: sechster Schritt
- 107: siebter Schritt

## Patentansprüche

1. Hubvorrichtung (20) zur kontrollierten longitudinalen Bewegung einer Sonde (16) in einem Arbeitskanal (13) eines medizinischen Instruments (10) mit:
einem distalen Bauteil (50) mit einer Kopplungseinrichtung (60) zum Befestigen der Hubvorrichtung (20) an einem proximalen Ende (11) des Arbeitskanals (13) des medizinischen Instruments (10);
einem proximalen Bauteil (30) mit einer Halteeinrichtung (32, 34) zum Halten eines Abschnitts (17) der Sonde (16),
wobei das proximale Bauteil (30) relativ zu dem distalen Bauteil (50) linear verschiebbar ist und die Position der Halteeinrichtung (32, 34) relativ zur Kopplungseinrichtung (60) stufenlos einstellbar ist,
wobei die Hubvorrichtung (20) eine Verbindungseinrichtung (70) aufweist, mittels der das proximale Bauteil (30) mit dem distalen Bauteil (50) lösbar verbunden ist, wobei die Verbindungseinrichtung (70) einen ersten Zustand aufweist, in dem das proximale Bauteil (30) verdrehsicher am distalen Bauteil (50) festgelegt ist,
wobei die Verbindungseinrichtung (70) einen zweiten Zustand aufweist, in dem das proximale Bauteil (30) mit dem distalen Bauteil (50) verbunden und gegenüber diesem verdrehbar ist,
**dadurch gekennzeichnet, dass** die Verbindungseinrichtung (70) ferner einen dritten Zustand aufweist, in dem das proximale Bauteil (30) von dem distalen Bauteil (50) trennbar ist.

2. Hubvorrichtung (20) nach einem der vorangehenden Ansprüche, **dadurch gekennzeichnet, dass** die Verbindungseinrichtung (70) eine Schraubenmutter (71) umfasst.

3. Hubvorrichtung (20) nach einem der vorangehenden Ansprüche, wobei das distale Bauteil (50) ein erstes Rohr (51) umfasst,
das proximale Bauteil (30) ein zweites Rohr (31) umfasst und das erste Rohr (51) und das zweite Rohr (31) eine Teleskoprohranordnung bilden.

4. Hubvorrichtung (20) nach dem vorangehenden Anspruch, bei der das zweite Rohr (31) einen nicht-kreisförmigen Querschnitt aufweist, ferner mit:
einem Führungselement (72), das eine lineare Bewegung des zweiten Rohrs (31) relativ zum Führungselement (72) zulässt und eine Rotation des zweiten Rohrs (31) relativ zum Führungselement (72) formschlüssig unterbindet, wobei das Führungselement (72) mit dem distalen Bauteil (50) mechanisch verbunden oder verbindbar ist.

5. Hubvorrichtung (20) nach dem vorangehenden Anspruch, bei der das Führungselement (72) bei festgezogener Schraubenmutter (71) formschlüssig an einer Rotation gegenüber dem distalen Bauteil (50) gehindert ist.

6. Hubvorrichtung nach Anspruch 3, bei der das erste Rohr einen nicht-kreisförmigen Querschnitt aufweist, ferner mit:
einem Führungselement, das eine lineare Bewegung des ersten Rohrs relativ zum Führungselement zulässt und eine Rotation des ersten Rohrs relativ zum Führungselement formschlüssig unterbindet, wobei das Führungselement mit dem proximalen Bauteil verbunden oder verbindbar ist.

7. Hubvorrichtung (20) nach dem vorangehenden Anspruch, bei der das Führungselement (72) bei festgezogener Schraubenmutter (71) formschlüssig an einer Rotation gegenüber dem proximalen Bauteil (30) gehindert ist.

8. Hubvorrichtung (20) nach Anspruch 3 oder 4, ferner mit einem ringförmigen Reibelement (39) zwischen dem ersten Rohr (51) und dem zweiten Rohr (31), zur Erzeugung einer Reibungskraft bei Verschiebung des proximalen Bauteils (30) relativ zum distalen Bauteil (50).

9. Hubvorrichtung (20) nach einem der vorangehenden Anspruche, bei der die Halteeinrichtung wenigstens ein Dichtungselement (32) und eine Kompressionseinrichtung (34) zur Kompression des Dichtungselements (32) umfasst.

10. Hubvorrichtung (20) nach dem vorangehenden Anspruch, bei der die Kompressionseinrichtung (34) ein Schraubgewinde (36, 42) umfasst.

11. Hubvorrichtung (20) nach einem der vorangehenden Ansprüche, wobei die Halteeinrichtung (32, 34) ferner zum fluiddichten Verschließen des proximalen Endes (21) der Hubvorrichtung (20) ausgebildet ist.

12. Hubvorrichtung (20) nach einem der vorangehenden Ansprüche, wobei die Hubvorrichtung (20) zur kontrollierten Bewegung eines proximalen Abschnitts (17) eines Lichtwellenleiters (16) ausgebildet ist.

13. Hubvorrichtung (20) nach einem der vorangehenden Ansprüche, **dadurch gekennzeichnet, dass**
sie mit dem medizinischen Instrument (10) verbunden ist und die Kopplungseinrichtung (60) eine nicht-runde distale Aufnahme (68) aufweist, so dass die Hubvorrichtung (20) verdrehsicher auf einem zur nicht-runden Aufnahme (68) korrespondierenden Ausgang (14) des Arbeitskanals (11) gelagert ist.

## Claims

1. Lifting device (20) for controlled longitudinal movement of a probe (16) in a working channel (13) of a medical instrument (10), having:
a distal component (50) with a coupling mechanism (60) for fastening the lifting device (20) to a proximal end (11) of the working channel (13) of the medical instrument (10);
a proximal component (30) with a holding mechanism (32, 34) for holding a portion (17) of the probe (16),
wherein the proximal component (30) is movable linearly with respect to the distal component (50), and the position of the holding mechanism (32, 34) relative to the coupling mechanism (60) is adjustable in a stepless manner,
wherein the lifting device (20) has a connecting mechanism (70), by means of which the proximal component (30) is connected to the distal component (50) in a releasable manner, wherein the connecting mechanism (70) has a first state, in which the proximal component (30) is fastened to the distal component (50) in a manner secure against rotation,
wherein the connecting mechanism (70) has a second state, in which the proximal component (30) is connected to the distal component (50) and is rotatable relative thereto,
**characterized in that** the connecting mechanism (70) moreover has a third state, in which the proximal component (30) is separable from the distal component (50).

2. Lifting device (20) according to the preceding claim, **characterized in that** the connecting mechanism (70) comprises a screw nut (71).

3. Lifting device (20) according to one of the preceding claims, wherein the distal component (50) comprises a first tube (51), the proximal component (30) comprises a second tube (31), and the first tube (51) and the second tube (31) form a telescopic tube arrangement.

4. Lifting device (20) according to the preceding claim, in which the second tube (31) has a non-circular cross section, also with:
a guide element (72), which permits a linear movement of the second tube (31) relative to the guide element (72) and, with a form fit, suppresses a rotation of the second tube (31) relative to the guide element (72), wherein the guide element (72) is mechanically connected or connectable to the distal component (50).

5. Lifting device (20) according to the preceding claim, in which, when the screw nut (71) is tightened, the guide element (72) is prevented from rotation with respect to the distal component (50) by means of a form fit.

6. Lifting device according to Claim 3, in which the first tube has a non-circular cross section, also with:
a guide element, which permits a linear movement of the first tube relative to the guide element and, with a form fit, suppresses a rotation of the first tube relative to the guide element, wherein the guide element is connected or connectable to the proximal component.

7. Lifting device (20) according to the preceding claim, in which, when the screw nut (71) is tightened, the guide element (72) is prevented from rotation with respect to the proximal component (30) by means of a form fit.

8. Lifting device (20) according to Claim 3 or 4, also with:
an annular friction element (39) between the first tube (51) and the second tube (31), for generating a frictional force during movement of the proximal component (30) relative to the distal component (50).

9. Lifting device (20) according to one of the preceding claims, in which the holding mechanism comprises at least one sealing element (32) and a compression mechanism (34) for compressing the sealing element (32).

10. Lifting device (20) according to the preceding claim, in which the compression mechanism (34) comprises a screw thread (36, 42).

11. Lifting device (20) according to one of the preceding claims, wherein the holding mechanism (32, 34) is further designed to close the proximal end (21) of the lifting device (20) in a fluid-tight manner.

12. Lifting device (20) according to one of the preceding claims, wherein the lifting device (20) is designed for controlled movement of a proximal portion (17) of an optical waveguide (16).

13. Lifting device (20) according to one of the preceding claims, **characterized in that**
it is connected to the medical instrument (10), and the coupling mechanism (60) has a non-round distal seat (68), such that the lifting device (20) is mounted, in a manner secure against rotation, on an outlet (14) of the working channel (13), which outlet (14) corresponds to the non-round seat (68).

## Revendications

1. Dispositif de levage (20) pour le déplacement longitudinal contrôlé d'une sonde (16) dans un canal de travail (13) d'un instrument médical (10), comprenant :
un composant distal (50) avec un dispositif d'accouplement (60) pour fixer le dispositif de levage (20) à une extrémité proximale (11) du canal de travail (13) de l'instrument médical (10) ;
un composant proximal (30) avec un dispositif de retenue (32, 34) pour retenir une portion (17) de la sonde (16),
le composant proximal (30) pouvant être déplacé linéairement par rapport au composant distal (50) et la position du dispositif de retenue (32, 34) par rapport au dispositif d'accouplement (60) pouvant être ajustée de manière continue,
le dispositif de levage (20) présentant un dispositif de connexion (70) au moyen duquel le composant proximal (30) est connecté de manière amovible au composant distal (50), le dispositif de connexion (70) présentant un premier état dans lequel le composant proximal (30) est fixé de manière solidaire en rotation au composant distal (50),
le dispositif de connexion (70) présentant un deuxième état dans lequel le composant proximal (30) est connecté au composant distal (50) et peut tourner par rapport à celui-ci,
**caractérisé en ce que** le dispositif de connexion (70) présente en outre un troisième état dans lequel le composant proximal (30) peut être séparé du composant distal (50).

2. Dispositif de levage (20) selon la revendication précédente, **caractérisé en ce que** le dispositif de connexion (70) comprend un écrou fileté (71).

3. Dispositif de levage (20) selon l'une quelconque des revendications précédentes, dans lequel le composant distal (50) comprend un premier tube (51),
le composant proximal (30) comprend un deuxième tube (31) et le premier tube (51) et le deuxième tube (31) forment un agencement de tubes télescopiques.

4. Dispositif de levage (20) selon la revendication précédente, dans lequel le deuxième tube (31) présente une section transversale non circulaire, et comprenant en outre :
un élément de guidage (72) qui permet un mouvement linéaire du deuxième tube (31) par rapport à l'élément de guidage (72) et qui empêche, par engagement par correspondance de forme, une rotation du deuxième tube (31) par rapport à l'élément de guidage (72), l'élément de guidage (72) étant connecté ou pouvant être connecté mécaniquement au composant distal (50).

5. Dispositif de levage (20) selon la revendication précédente, dans lequel l'élément de guidage (72), lorsque l'écrou fileté (71) est vissé, est empêché, par un engagement par correspondance de forme, de tourner par rapport au composant distal (50).

6. Dispositif de levage selon la revendication 3, dans lequel le premier tube présente une section transversale non circulaire, et comprenant en outre :
un élément de guidage qui permet un mouvement linéaire du premier tube par rapport à l'élément de guidage et qui empêche, par engagement par correspondance de forme, une rotation du premier tube par rapport à l'élément de guidage, l'élément de guidage étant connecté ou pouvant être connecté au composant proximal.

7. Dispositif de levage (20) selon la revendication précédente, dans lequel l'élément de guidage (72), lorsque l'écrou fileté (71) est vissé, est empêché, par engagement par correspondance de forme, de tourner par rapport au composant proximal (30).

8. Dispositif de levage (20) selon la revendication 3 ou 4, comprenant en outre
un élément de friction annulaire (39) entre le premier tube (51) et le deuxième tube (31), afin de générer une force de friction lors du déplacement du composant proximal (30) par rapport au composant distal (50).

9. Dispositif de levage (20) selon l'une quelconque des revendications précédentes, dans lequel le dispositif de retenue comprend au moins un élément de joint d'étanchéité (32) et un dispositif de compression (34) pour la compression de l'élément de joint d'étanchéité (32).

10. Dispositif de levage (20) selon la revendication précédente, dans lequel le dispositif de compression (34) comprend un filetage de vis (36, 42).

11. Dispositif de levage (20) selon l'une quelconque des revendications précédentes, dans lequel le dispositif de retenue (32, 34) est en outre réalisé pour assurer la fermeture étanche aux fluides de l'extrémité proximale (21) du dispositif de levage (20).

12. Dispositif de levage (20) selon l'une quelconque des revendications précédentes, dans lequel le dispositif de levage (20) est réalisé pour permettre le mouvement contrôlé d'une portion proximale (17) d'un guide d'onde optique (16).

13. Dispositif de levage (20) selon l'une quelconque des revendications précédentes, **caractérisé en ce qu'**il est connecté à l'instrument médical (10) et le dispositif d'accouplement (60) présente un logement distal non circulaire (68) de sorte que le dispositif de levage (20) soit monté de manière solidaire en rotation sur une sortie (14) du canal de travail (13) correspondant à un logement non circulaire (68).
